# EUROPÄISCHE PATENTANMELDUNG

(11) **EP 1 138 285 A1**
(43) Veröffentlichungstag der Anmeldung: **04.10.2001**
(21) Anmeldenummer: 01810261.6
(22) Anmeldetag: 15.03.2001
(51) Int. Cl.: A61F 2/44, A61F 2/46, A61L 27/58

(54) **Wirbelkäfig zum Einbau zwischen zwei Wirbelkörper der Wirbelsäule**

(30) Priorität: 29.03.2000 CH 606002000
(71) Anmelder: Implant Design AG, 8610 Uster (CH)
(72) Erfinder: Bühler, Markus, 8610 Uster (CH); Sgier, Friedrich, Dr., 6006 Luzern (CH)
(74) Vertreter: Felber, Josef

(57) **Zusammenfassung**

Der Stützkorb, in der Fachsprache Cage genannt, ist zum Einbau zwischen zwei Wirbel der Wirbelsäule bestimmt. In einer Ausführung als cervicaler Cage wir er zwischen zwei Halswirbel eingesetzt, als lumbaler Cage ausgeführt zwischen zwei Lendenwirbel. Er bildet einen geschlossenen rechteckigen Rahmen (1) mit innerem Freiraum (7) zur Aufnahme von Knochenersatzmaterial. Der Cage ist im Spritzverfahren aus bioresorbierbarem Poly(L-lactid-co-D₃L-lactid) der Formel (C₃H₄O₂)ₙ hergestellt. Er bleibt implantiert während ca. 20 Monaten hinreichend druckfest, und löst sich danach vollständig auf, sodass eine bisher allfällige Entfernung des Implantates in jedem Fall hinfällig wird.

## Beschreibung

Die vorliegende Erfindung betrifft einen medizinischen Stützkorb, wie er für Operationen an der Wirbelsäule benötigt wird. Bei sogenannten Wirbelsäulen- und Bandscheibenschäden oder Bandscheibenvorfällen infolge etwa eines Unfalls wird bis anhin mit temporären oder permanenten Implantaten gearbeitet. Als Werkstoffe werden Polyetheretherketone (PEEK) und Titan eingesetzt. Ein solcher Stützkorb oder Stützrahmen, in der Fachsprache Cage genannt, hat die Aufgabe, nach einer Beschädigung der Wirbel und/oder Bandscheiben einen definierten Abstand zwischen zwei Wirbelkörpern einzuhalten. Je nach dem Einbauort spricht man von einem cervicalen Cage, wenn er im Halswirbelbereich eingesetzt wird, und von lumbalen Cages, wenn diese im Lendenbereich der Wirbelsäule zum Einsatz kommen. Die Cages bilden im Falle von cervicalen Cages einen viereckigen, annähernd quadratischen Rahmen mit allseits leicht abgerundeten Ecken. Der Rahmen misst ca. 13mm in der Breite und ca. 15mm in der Länge und weist eine Höhe von 5.5mm bis 7.0mm auf. Die Rahmenwände sind ca. 2mm stark, sodass ein etwa quadratischer Raum frei bleibt. Die lumbalen Cages hingegen bilden einen rechteckförmig in die Länge gezogenen Rahmen, der an einem Ende über etwa einen Drittel der Rahmenlänge zur Seite hin gebogen ist, wobei der Rahmen an diesem Ende anstelle von zwei Ecken und einem diese verbindenden ebenen Stück praktisch kreisrund geformt ist. Sowohl bei den cervicalen wie auch bei den lumbalen Cages sind deren Oberseiten in einer Richtung leicht bombiert und weisen quer dazu verlaufende Rippen auf. Die ebene Unterseite des cervicalen Cage sowie auch die leicht bombierte Unterseite des lumbalen Cage weist ebensolche, in der gleichen Richtung verlaufende Rippen auf. Alle anderen Oberflächen sind glatt ausgeführt. Gegen ihre Hinterseite hin, das heisst gegen diejenige Seite hin, mit welcher die Cages voran zwischen zwei Wirbel geschoben werden, sind sie insgesamt leicht keilförmig zulaufend geformt. Der frei bleibende Raum im Innern dieser Cages wird dann mit Eigenspongiosa oder tierischen Matrizen gefüllt, um ein inneres knochenartiges Verwachsen der Wirbelkörper zu erreichen. Ein solcher Cage dient somit erstens als vorübergehendes oder permanentes Stützelement und zweitens als Träger oder Korb (Cage) für das biologische Material, bis nach seiner Implantierung über eine gewisse Zeitdauer eine Fusion der beiden voneinander beabstandet gehaltenen Wirbelkörper durch Knochenmaterial entstanden ist. Dieser Prozess wird als cervicale oder lumbale Spondylodese bezeichnet.

Zum Implantieren eines Cages wird dem Patienten unter Vollnarkose in Rückenlage die Wirbelsäule an der zu behandelnden Stelle von vorne freigelegt und das geschädigte Material zwischen zwei Wirbeln mit einer Kürette entnommen. Die Knochenplatten sollten dabei möglichst sauber vorbereitet werden. Hernach wird für jeden zu setzenden Cage zunächst ein Probecage mit glatter Oberund Unterseite mit einer speziellen Zange von vorne ergriffen und zwischen die Wirbel geschoben und es wird geprüft, ob dieser Probecage passgenau sitzt. Mittels solcher Probecages verschiedener Grösse wird die richtige Dimension jedes einzusetzenden Cage ermittelt. Steht fest, welcher Cage oder welche Cages eingesetzt werden, so wird jeder solche Cage in eine spezielle Halterung eingelegt und sein innerer Freiraum wird mit Knochenersatzmaterial oder einem autolytischen, das heisst selbst auflösenden Implantat gefüllt, wobei dieses dicht in den Freiraum gestopft wird. Der so befüllte Cage wird sodann mit einer speziellen Zange, mittels welcher er von vorne an eigens am Cage vorgesehenen Grifflöchern ergriffen werden kann, zwischen die zwei gegeneinander abzustützenden Wirbel geschoben. Die Wirbel werden dabei leicht auseinandergeschoben und der passgenau eingesetzte Cage wird hernach dank seiner gerippten Ober- und Unterseite sicher zwischen den zwei Wirbeln gehalten. Die Griffzange kann hernach abgesetzt und entfernt werden. Ein derart implantierter Cage stellt sofort nach der Operation die Stabilität der Wirbelsäule im behandelten Bereich sicher. In einigen Fällen wird der Cage mit spezieller Platten zusätzlich in seiner Lage gesichert. Dann wird die Wunde wieder geschlossen. Nur beim Auftreten von Komplikationen, etwa infolge ungenügenden Durchbaus des Cage, ist eine Revision nötig, um den instabilen Cage zu entfernen. Ein nicht genügend durchbauter und anhaltend instabiler Cage stellt nämlich infolge von Lageänderungen (Luxation) eine Gefahr für die Dura und den Oesophagus dar. Eine Instabilität hat auch Mikrobewegungen zwischen dem Implantat und dem anliegenden Knochen bzw. Knochenmaterial zur Folge, was lokal zu einer Knochenresorption führen kann. Je nach Art des verarbeiteten biologischen Materials dauert der Durchbau mehr oder weniger lange. Bei Verwendung von Eigenspongiosa aus dem Beckenkamm, also körpereigenem organischen Material, muss mit mindestens 3 Monaten bis hin zu 10 Monaten gerechnet werden, bei körperfremden Collagen-Matrizen oder Trikalziumphosphat, d.h. künstlicher erzeugter Knochensubstanz, dauert das Gleiche ca. 6 bis 12 Monate.

Es versteht sich, dass es eine grosse Erleichterung darstellen würde, sowohl für den Patienten einerseits wie auch andrerseits in bezug auf die implizierten Operationskosten, wenn sich ein Entfernen des Implantates in jedem Fall erübrigen würde. Weiter ist es wünschenswert, dass auch der Raum, der zunächst vom Cage eingenommen wird, irgendeinmal wieder von körpereigenem Material gefüllt würde. Das ist aber nur denkbar, wenn das Implantat sich nach der geforderten Zeit von ca. 12 Monaten von selbst auflösen würde, also vom Körper resorbiert würde, und zweitens, wenn das Implantat über die geforderte Zeitperiode von 12 Monaten hinreichend stabil bliebe, um die Tragfunktion zu übernehmen. Es stellt sich also ein ähnliches Problem wie wenn man einen Zuckerwürfel leicht in Wasser stehend mit Druck beaufschlagen würde. Er hält das eine gewisse Zeit aus, dann nimmt seine Tragfähigkeit mit zunehmender Aufnahme von Wasser ab, schliesslich nimmt die Tragfähigkeit abrupt ab und er zerfällt und löst sich vollständig im Wasser auf. Die Problematik bei der Realisierung eines derartigen sich resorbierenden Cage ist darin zu sehen, dass damit nicht am menschlichen Körper herumgeprobt werden kann, weil solche Versuche im Wirbelsäulenbereich viel zu heikel und zu gefährlich wären. Es musste daher ein Weg gefunden werden, um das geforderte Pflichtenheft durch Verschmelzen von Erkenntnissen in verschiedenen Disziplinen rein technisch zu erfüllen.

Die Aufgabe der vorliegenden Erfindung ist es daher, die technischen Spezifikationen für einen Cage anzugeben, sodass dieser nach dem Implantieren erstens hinreichend lange seine Druckbelastbarkeit erhält, und zweitens danach zuverlässig vom Körper resorbierbar ist.

Diese Aufgabe wird gelöst von einem medizinischer Stützkorb zum Einbau zwischen zwei Wirbel der Wirbelsäule, welcher einen geschlossenen Rahmen mit innerem Freiraum zur Aufnahme von Knochenersatzmaterial bildet, wobei er sich dadurch auszeichnet, dass er aus bioresorbierbarem Poly(L-lactid-co-D₃L-lactid) der Formel (C₃H₄O₂)ₙ hergestellt ist.

In den Figuren ist ein derartiger cervicaler Stützkorb und ein lumbaler Stützkorb dargestellt, wobei die Figuren zeigen:
- Figur 1 :: Eine perspektivische Ansicht eines cervicalen Stützkorbes;
- Figur 2 :: Eine Seitenansicht des cervicalen Stützkorbes;
- Figur 3 :: Einen Querschnitt längs durch den cervicalen Stützkorb;
- Figur 4 :: Eine perspektivische Ansicht eines lumbalen Stützkorbes.

Zunächst einige weitere Ausführungen zum Baustoff: Poly(L-lactid-co-D₃L-lactid) wird zum Beispiel von Boehringer Ingelheim Pharma KG, BA Chemicals, D-55216 Ingelheim am Rhein, Deutschland, hergestellt und unter der Bezeichnung D.L-Lactyd Resomer® LR 708 verkauft. Es handelt sich um ein fast weisses und fast geruchloses Granulat. Die polymere Zusammensetzung liegt in einem Verhältnis von 67:33 bis 73:27 des molekularen Verhältnisses zwischen L-lactid: D₃L-lactid. Hier sind die wichtigsten Charakteristika dieses Materials LR 708 anhand einer Tabelle ersichtlich:

| | | | |
|---|---|---|---|
| Verarbeitungstemperatur [°C]¹⁾ | T = 245°C | T = 260°C | T = 275°C |
| Zugfestigkeit σ_{zM} [MPa]²⁾³⁾ | 79 | 72 | 60 |
| Dehnung bei Maximalspannung ε_{zM} [%] ²⁾³⁾ | 3.11 | 2.77 | 2.48 |
| Reißspannung σ_{zR} [MPa] ²⁾³⁾ | 71.4 | 57.52 | 52.72 |
| Reißdehnung ε_{zR} [MPa] ²⁾³⁾ | 6.03 | 7.37 | 2.91 |
| Bindenahtfestigkeit σ_{BzM} [MPa]²⁾³⁾ | 65 | 68 | 60 |
| Zug-E-Modul E [MPa] ²⁾⁴⁾ | 660 | 660 | 660 |
| Biegespannung σ_{B} [Mpa] ²⁾³⁾ | 104.3 | 101 | 65.6 |
| Randfaserdehnung ε_{B} [%] ²⁾³⁾ | 4.37 | 4.29 | 4.25 |
| Biegespannung (Bindenaht) σ_{BB} [MPa]²⁾³⁾ | 98 | 99.5 | 80 |
| Randfaserdehnung (Bindenaht) ²⁾³⁾ | 4.53 | 4.24 | 3.93 |
| Kerbschlagzähigkeit [J]²⁾ | 0.23 | 0.32 | 0.25 |
| Schrumpf [%]⁵⁾ | 28 | 26 | 14 |
| Inhärente Viskosität [dl/g] | 2.9 | 2.4 | 1.6 |

| | | | |
|---|---|---|---|
| ¹⁾ Fertigungsparameter: Einspritzvolumenstrom Q = 30 [cm³/s] Werkzeugwandtemperatur T_{W} = 15[°C] | | | |
| ²⁾ Prüftemperatur TP = 37[°C] | | | |
| ³⁾ Prüfgeschwindigkeit VP = 50 [mm/min] | | | |
| ⁴⁾ Prüfgeschwindigkeit VP = 1 [mm/min] | | | |
| ⁵⁾ bei 70°C; 12h ⁶⁾ bei 5kg Belastung; 1mm Düse | | | |

Weitere für den Einsatz als Werkstoff für cervicale und lumbale Cages wichtige Eigenschaften sind folgende:

| | |
|---|---|
| Trocknungstemperatur [°C] | ≤ 90 Minuten oder weniger |
| Trocknungsmethode | Adsorptionsumlufttrockner / Vakuumtrockner |
| Minimale Trocknungszeit | 4 Stunden |
| Ideale Trocknungszeit | 8 Stunden |
| Glasübergangstemperatur T_{G} [°C] | 55 |
| Kristallisationstemperatur T_{K} [°C] | 110 |
| Inhärente Viskosität [dl/g] | ca. 5.9 |
| Mark-Houwing-Konstanten | K = 5.45 * 10⁻⁴; α = 0.73 nach Tsuruta |
| Dichte bei Raumtemperatur [g/cm³] | P = 1.259 |
| Spez. Wärmekapazität [kJ/kmol K] | Cₚ = 2.564 |

Mit diesem grundsätzlich bioresorbierbaren Material wurden im Hinblick auf die Herstellung sowohl cervicaler wie auch lumbaler Cages umfangreiche physikalische Versuche angestellt. Es zeigte sich, dass D.L-Lactyd Resomer® LR 708 eine Druckfestigkeit von ca. 120 N/mm² erbrachte, während für einen cervicalen Cage mindestens 40 N/mm² und für einen lumbalen Cage mindestens 80 N/mm² gefordert werden. Die Bioresorption resultiert erst nach ca. 20 Monaten in einem Masseverlust, das heisst, innerhalb von 20 Monaten bleibt die Belastbarkeit annähernd unverändert, während für ein Cage-Implantat maximal 12 Monate gefordert werden. Danach setzt sich die Bioresorption über 10 Monate fort, bis schliesslich das Material komplett aufgelöst ist und der Cage also verschwunden ist. Das Material kann im Spritzverfahren verarbeitet werden und eignet sich daher vorzüglich zur Herstellung eines sowohl cervicalen wie auch lumbalen Cages.

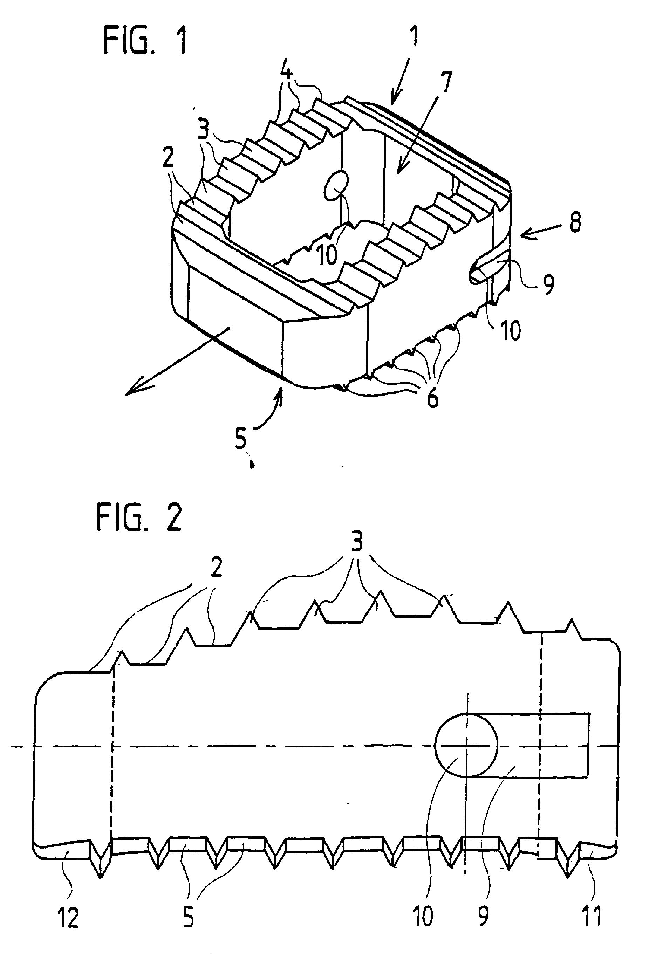

In Figur 1 ist nun zunächst ein solcher cervicaler Stützkorb (Cage), der aus dem Material D.L-Lactyd Resomer® LR 708 gespritzt wird, in perspektivischer Darstellung gezeigt. Im wesentlichen bildet dieser Stützkorb, in der Fachsprache Cage genannt, einen viereckigen Rahmen 1. Alle äusseren und inneren Ecken sind zunächst etwas abgeschrägt und dann die Kanten abgerundet. Man sieht den Cage hier mit seinem hinteren Ende in der Front dargestellt, während seine Vorderseite nach hinten gewandt dargestellt ist. Er wird nämlich in der mit dem Pfeil angedeuteten Richtung von der Vorderseite der Wirbelsäule her in diese hineingeschoben. Die Oberseite des Cage ist von dessen vorderer nach dessen hinterer Seite hin leicht bombiert und fällt zudem gegen seine Hinterseite hin leicht ab, sodass der Cage insgesamt leicht keilförmig ist. Auf der Oberseite 2 verlaufen quer zur Richtung der Bombierung Rippen 3 mit dreieckförmigem Querschnitt und einer scharfen oberen Kante 4, wobei die Rippenhöhe max. 0.7mm misst. Gleichermassen sind auch auf der insgesamt ebenen Unterseite 5 des Cage solche Rippen 6 angeformt. Im Innern bildet der Cage einen Freiraum 7, welcher zur Aufnahme des Knochenersatzmaterials dient. Im vorderen Teil 8 des Cage weist dieser auf beiden Seiten horizontal verlaufende Nuten 9 auf, welche an ihrem Ende in je ein Loch 10 münden. Diese Nuten 9 und Löcher 10 dienen dazu, dass der Cage von vorne mittels einer speziellen Griffzange mit zwei zueinander gerichteten Zapfen, welche in die Löcher 10 einpassen, ergriffen werden kann, und dann in jeder Richtung stabil vom Chirurgen gehalten werden kann.

Die Figur 2 zeigt eine Seitenansicht des Stützkorbes. Man sieht hier sehr schön die bombierte Oberseite und die quer zur Krümmung verlaufenden Rippen 3, die hier mit ihrer Kante einen spitzen Winkel von 60° einschliessen. In vorderen, hier rechts in der Zeichnung gezeigten Bereich des Cage sieht man das Loch 10 für den Zapfen der Griffzange, sowie die Nut 9, in welche der Zangenarm, der den Zapfen trägt, einpasst, sodass der Cage in jeder Hinsicht fest und dennoch ausschliesslich von vorne gehalten werden kann. Die Unterseite 5 des Cage ist im wesentlichen eben ausgeführt, wenngleich sie vorne und hinten leicht abfällt, wie das anhand der Bereiche 11 vorne und 12 hinten gesehen werden kann. Die Rippen 6 sind in gleicher Weise ausgeführt wie jene auf der Oberseite.

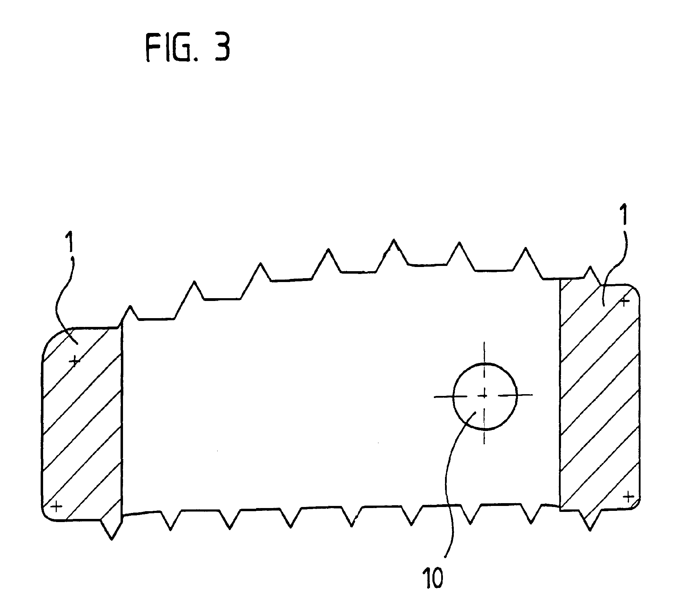

Die Figur 3 zeigt einen Querschnitt längs durch den Stützkorb bzw. den Cage. Anhand dieses Schnittes ersieht man die Stärke des Rahmens 1, der überall etwa 2mm stark ist, sowie das Loch 10 zur Aufnahme des Zapfens eines Zangenarmes.

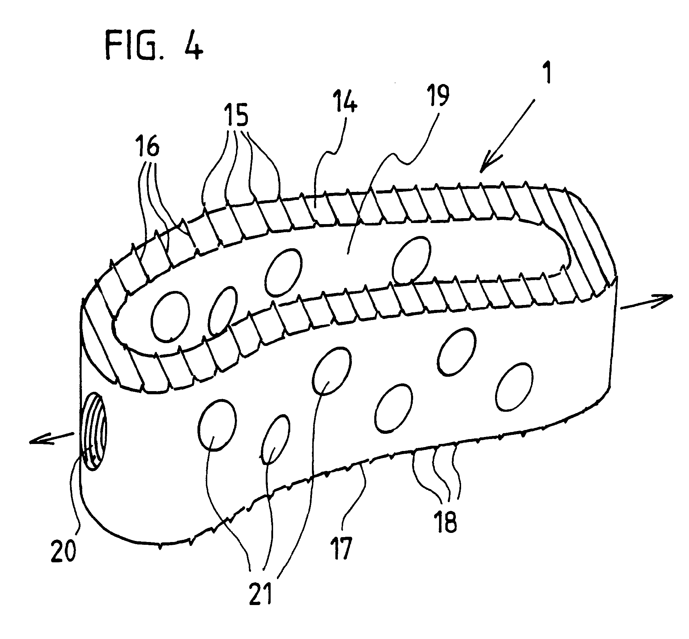

Die Figur 4 zeigt eine alternative Ausführung als lumbaler Cage, der also zum Einbau zwischen zwei Lendenwirbel dient. Für diesen Wirbelsäulenbereich werden jeweils zwei der hier gezeigten Cages achsensymmetrisch nebeneinander zwischen zwei Wirbel eingebaut. Dieser lumbale Stützkorb (Cage) ist wiederum aus dem Material D.L-Lactyd Resomer® LR 708 gespritzt. Im wesentlichen bildet auch dieser Stützkorb einen geschlossenen Rahmen 1. Alle äusseren und inneren Ecken sind abgerundet. Man sieht den Cage hier mit seinem vorderen Ende in der Front dargestellt, während seine Hinterseite nach hinten gewandt dargestellt ist, also gerade umgekehrt wie der Cage in Figur 1 dargestellt ist. Er wird in der mit den Pfeilen angedeuteten Richtung entweder von der Vorderseite der Wirbelsäule her, also ventral zwischen zwei Lendenwirbel hineingeschoben, oder aber bei Bedarf auch von der Hinterseite der Wirbelsäule her. Dieser Cage weist eine Grundfläche von maximal 25mm x 12mm auf, und eine Höhe von zwischen 6.0mm bis 14.0mm, wobei der Rahmen 1 auf einer Seite über 2/3 der Länge in ein rechteckiges Ende ausläuft und auf der anderen Seite über 1/3 der Länge in eine seitlich abgebogene Schlaufe ausläuft. Die Oberseite des Cage ist von dessen vorderer nach dessen hinterer Seite hin leicht bombiert und fällt zudem gegen seine Hinterseite hin leicht ab, sodass der Cage insgesamt leicht keilförmig ist. Auf der Oberseite 14 verlaufen quer zur Richtung der Bombierung Rippen 15 mit dreieckförmigem Querschnitt und einer scharfen oberen Kante 16, wobei die Rippenhöhe max. 0.7mm misst. Gleichermassen sind auch auf der ebenfalls nach aussen bombierten Unterseite 17 des Cage solche Rippen 18 angeformt. Im Innern bildet der Cage einen Freiraum 19, welcher zur Aufnahme des Knochenersatzmaterials dient. Vorne und auch hinten, dort freilich nicht einsehbar, weist der Cage je eine durchgehende Gewindebohrung 20 auf. Diese Gewindebohrungen 20 dienen dazu, dass der Cage von vorne oder von hinten mittels eines speziellen Werkzeuges gehalten werden kann. Das Werkzeug weist einen Gewindezapfen auf, der in die entsprechende Gewindebohrung 20 eingeschraubt wird, wonach der Chirurg mit dem Werkzeug den Cage sicher führen und halten kann. Nach der Positionierung zwischen den Lendenwirbeln wird das Werkzeug wieder aus der Gewindebohrung 20 herausgeschraubt und zurückgezogen. In den Seitenwänden dieses lumbalen Cage sind eine Anzahl Löcher 21 vorhanden. Diese dienen dazu, dass sich Knochenmaterialbrücken zwischen dem Raum ausserhalb und innerhalb des Cage bilden können und somit der Cage mit der Zeit innig mit der Umgebung verwächst, bis er sich anschliessend vollständig resorbiert.

Resorbierbare Cages bieten den grundsätzlichen Vorteil, dass der von ihnen zunächst eingenommene Raum nach der Resorption des Cage ebenfalls vollständig mit körpereigenem Material durchwachsen wird und somit eine maximale Tragkraft und Belastbarkeit der Wirbelsäule erreicht wird.

## Patentansprüche

1. Medizinischer Stützkorb zum Einbau zwischen zwei Wirbel der Wirbelsäule, welcher einen geschlossenen Rahmen (1) mit innerem Freiraum (7;19) zur Aufnahme von Knochenersatzmaterial bildet, wobei er sich dadurch auszeichnet, dass er aus bioresorbierbarem Poly(L-lactid-co-D₃L-lactid) der Formel (C₃H₄O₂)ₙ hergestellt ist.

2. Medizinischer Stützkorb nach Anspruch 1, **dadurch gekennzeichnet, dass** er als cervicaler Stützkorb (cervicaler Cage) zum Einbau zwischen zwei Halswirbel ausgeführt ist und eine Grundfläche von maximal 15mm x 13mm aufweist, und eine Höhe von zwischen 5.5mm bis 7.0mm, sowie **dass** seine Oberseite von vorne nach hinten bombiert ist und gegen die Hinterseite des Cage hin abfällt, sodass der Cage in Einbaurichtung insgesamt keilförmig zuläuft, sowie **dass** auf der Oberseite (2) quer zur Richtung der Bombierung Rippen (3) mit dreieckförmigem Querschnitt und einer scharfen oberen Kante (4) verlaufen, wobei die Rippenhöhe max. 0.7mm misst.

3. Medizinischer cervicaler Stützkorb nach einem der vorhergehenden Ansprüche, **dadurch gekennzeichnet, dass** im vorderen Bereich des Cages zwei horizontal verlaufende, einander gegenüberliegende Löcher (10) vorhanden sind, welche in horizontal nach vorne verlaufende Nuten münden, die sich rechtwinklig zu den Löchern (10) erstrecken, und **dass** die gegenüberliegenden Löcher (10) auf einer gemeinsamen Achse liegen.

4. Medizinischer Stützkorb nach Anspruch 1, **dadurch gekennzeichnet, dass** er als lumbaler Stützkorb (lumbaler Cage) zum Einbau zwischen zwei Lendenwirbel ausgeführt ist und eine Grundfläche von maximal 25mm x 12mm aufweist, und eine Höhe von zwischen 6.0mm bis 14.0mm, wobei der Rahmen (1) auf einer Seite über 2/3 der Länge in eine rechteckiges Ende ausläuft, und auf der anderen Seite über 1/3 der Länge in eine seitlich abgebogene Schlaufe ausläuft, sowie **dass** seine Oberseite von vorne nach hinten bombiert ist und gegen die Hinterseite des Cage hin abfällt, sodass der Cage in Einbaurichtung insgesamt keilförmig zuläuft, sowie **dass** auf der Oberseite (14) quer zur Richtung der Bombierung Rippen (15) mit dreieckförmigem Querschnitt und einer scharfen oberen Kante (16) verlaufen, wobei die Rippenhöhe max. 0.7mm misst.

5. Medizinischer lumbaler Stützkorb nach Anspruch 4, **dadurch gekennzeichnet, dass** er vorne und hinten je eine horizontal verlaufende Gewindebohrung (20) zum Einschrauben des Gewindezapfens eines Werkzeuges aufweist.

6. Medizinischer lumbaler Stützkorb einem der Ansprüche 4 bis 5, **dadurch gekennzeichnet, dass** seine Seitenwände von einer Anzahl horizontaler Löcher (21) durchsetzt sind.
